# EUROPÄISCHE PATENTSCHRIFT

(11) **EP 2 868 209 B1**
(45) Veröffentlichungstag und Bekanntmachung des Hinweises auf die Patenterteilung: **03.04.2019**
(21) Anmeldenummer: 13191008.5
(22) Anmeldetag: 31.10.2013
(51) Int. Cl.: A23L 9/10, A23L 27/30, A23L 2/60, A23L 27/00, A23G 4/10, A23G 4/06, A61Q 11/00, A23G 3/36, A23C 9/13, A23C 9/154, A23G 3/42, A61K 8/60, A61K 8/73, A23L 27/20

(54) **Stoffgemische**
Compound mixtures
Mélange de substances

(43) Veröffentlichungstag der Anmeldung: 06.05.2015
(73) Patentinhaber: Symrise AG, 37603 Holzminden (DE)
(72) Erfinder: Schäfer, Uwe, 31868 Ottenstein (DE); Krammer, Gerhard, 37603 Holzminden (DE); Glaubitz, Gerald, 37671 Höxter (DE); Riess, Thomas, 37603 Holzminden (DE); Ley, Jakob, 37603 Holzminden (DE); Paetz, Susanne, 37671 Höxter (DE)
(74) Vertreter: Eisenführ Speiser

(56) Entgegenhaltungen:
- EP-A1- 2 215 914
- EP-A1- 2 340 719
- EP-A1- 2 359 702
- EP-A1- 2 529 633
- EP-A1- 2 628 398
- US-A1- 2010 166 679
- US-A1- 2011 076 239

## Beschreibung

### GEBIET DER ERFINDUNG

Die Erfindung befindet sich auf dem Gebiet der Nahrungsmittel und betrifft und betrifft neue Stoffmischungen, die zum einen Aromastoffe mit verbesserter Süßungswirkung darstellen und dabei gleichzeitig die Löslichkeit anderer Aromastoffe in den konfektionierten Zubereitungen verbessern.

### STAND DER TECHNIK

Stevia wird aus den Blättern der Pflanze *Stevia rebaudiana* gewonnen und stellt ein komplexes Gemisch verschiedener Steviolglykoside dar. Steviaextrakte werden als kalorienfreie Süßungsmittel eingesetzt, wobei reines Stevia die 450fache Süßkraft von Zucker aufweist. Die Steviapflanze ist ursprünglich in Südamerika beheimatet, wo den Ureinwohnern ihre Süßkraft schon seit langem bekannt ist. Heutzutage wird die Pflanze in großem Maßstab in Japan und China angebaut. In der Europäischen Union ist Stevia seit 2011 als Lebensmittelzusatzstoffe E 960 zugelassen, auch wenn derzeit maximal 30 des Zuckers eines Produktes durch Stevia ersetzt werden darf.

Aus dem Stand der Technik sind Produkte, die Steviolglykoside als Süßungsmittel, so genannte "Sweetener" enthalten, hinreichend bekannt. Ebenfalls bekannt sind Unternehmungen, den Gehalt in Steviosiden durch Anreicherungsverfahren zu erhöhen und dabei einzelne Isomere anzureichern. So werden beispielsweise in den beiden internationalen Patentanmeldungen WO 2012 112180 A1, WO 2012 128775 A1 und WO 2012 129451 A1 (Pure Circle) Mischungen von Rebaudiosiden und Steviosiden sowie deren Verwendung als Sweetener beschrieben.

Gegenstand der WO 2013 096420 A1 (TCCC) ist ein chromatographisches Verfahren zur Aufreinigung von Steviolglykosiden.

Gegenstand der EP 2340719 A1 (SYMRISE) betrifft eine Aromazusammensetzung zum Verringern oder Unterdrücken eines unangenehmen (Geschmack-) Eindruckes in der Mundhöhle umfassend folgende Komponenten: (a) einen oder mehrere Süßungsmittel, z.B. unter anderen Steviolgylcoside, Stevioside, Steviolbioside, Rebaudiosides (z.B. Rebaudioside A, Rebaudioside B, Rebaudioside C, Rebaudioside D, Rebaudioside E, Rebaudioside F, Rebaudioside G, Rebaudioside H, Dulcoside, (b) ortho-Coumarinsäure, und optional(c1) ein oder mehrere bitter-maskierende Aromastoffe und/oder Duftstoffe, z.B. Hesperetin und optional (c2) eine oder mehrere zusätzliche Aromastoffe, wobei mindestens eine dieser Aromastoffe ein Süßungsverstärker Aromasubstanz ist, z.B. Phloretin, und optional (c3) eine oder mehrere inaktive Stoffe oder Trägerstoffe, wobei als bevorzugte Komponente Maltodextrin, Stärken, natürliche oder synthetische modifizierte Stärke oder Gummi arabicum unter anderen genannt werden.

Die EP 2359702 A1 (SYMRISE) betrifft ein Solubilisierungsmittel zum Solubilisieren von Polyphenolen, Flavonoiden und/oder Diterpenoidglucosiden in wässrigen Flüssigkeiten.

Dennoch zeichnen sich im Markt befindliche Steviaqualitäten dadurch aus, dass sie insgesamt einen bitteren, zuweilen adstringierenden Nachgeschmack aufweisen, was ihren breiten Einsatz nach wie vor erschwert. Auch die Löslichkeit von Steviolglykosiden in Nahrungsmitteln, speziell in flüssigen Produkten ist nicht zufriedenstellend

Die Aufgabe der vorliegenden Erfindung hat daher zum einen darin bestanden, das geschmacklich nicht einwandfreie sensorische Profil sowohl von Steviolglykosiden abzurunden. Zum anderen ist eine weitere Teilaufgabe darin zu sehen, gleichzeitig die vielfach unzureichende Löslichkeit von Aromastoffen im Allgemeinen und Steviolglykosiden im Besonderen in konfektionierten Endprodukten zu verbessern.

### BESCHREIBUNG DER ERFINDUNG

Gegenstand der Erfindung sind Stoffgemische, enthaltend
(a) Steviolglykoside,
(b) Stärkeabbauprodukte und
(c) einen oder mehrere phenolische süßverstärkenden Aromastoffe, ausgewählt aus der Gruppe, die gebildet wird von Hesperetin, Phloretin, 1-(2,4-Dihydroxy-phenyl)-3-(3-hydroxy-4-methoxy-phenyl)-propan-1-on, 7,3-Dihydroxy-4'methoxyflavan und 5-Hydroxy-4-(4-hydroxy-3-methoxy-phenyl)-7-methoxy-2-chromanon
wobei die Stoffgemische die Komponenten (a+b) und (c) im Gewichtsverhältnis 1:99 bis 99:1 enthalten.

Überraschenderweise wurde gefunden, dass die Mischungen bezogen auf die Komponenten (a) und (c) über eine verbesserte Süßungswirkung verfügen und in besonderer Weise geeignet sind, die geschmackliche Sensorik von Nahrungsmitteln abzurunden und zu optimieren. Insbesondere wird in der Kombination mit den Stärkeabbauprodukten und dem Hesperetin der bittere bis stechende Nachgeschmack maskiert. Gleichzeitig zeichnen sich die Mischungen nicht nur selbst durch eine verbesserte Löslichkeit aus, sie sind unerwarteterweise auch noch in der Lage, die Löslichkeit anderer Aromastoffe in den konfektionierten Endprodukten signifikant zu verbessern.

### STEVIOLGLYCOSIDE

Steviolglykoside werden aus der Pflanze *Stevia rebaudiana* ("Süßkraut", auch "Honigkraut") gewonnen und stellen ein Stoffgemisch dar, das als Süßstoff verwendet wird. Es besteht hauptsächlich Diterpenglykosiden wie dem Steviosid, Rebaudiosid A und sieben bis zehn weiteren Isomeren und Homologen, wie den Rebaudiosiden B, C und D und dem Steviolbiosid.

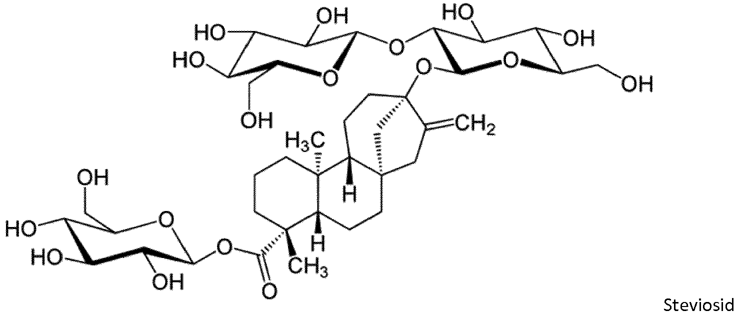

Die Anteile der enthaltenen Steviolglykoside unterscheiden sich nach Anbaugebiet und Pflanzensorte. Weitere Anteilsabweichungen entstehen, da Hersteller von Tafelsüße auf Steviolglykosid-Basis meist den Rebaudiosid-A-Anteil wegen des zuckerähnlichen Geschmacks erhöhen. Dem lakritzartigen Geschmack der Pflanze wird bei der Herstellung des Süßstoffgemisches durch Isolierung der süßenden Bestandteile und anschließender Komposition entgegengewirkt. Steviaprodukte können - als reines Rebaudiosid A - eine bis zu 450-fache Süßkraft von Zucker haben.

Steviolglykoside, die im Sinne der vorliegenden Erfindung bevorzugt sind, weisen mindestens 2, vorzugsweise mindestens 3 und insbesondere 2 bis 6 Glucoseeinheiten auf, wobei dabei mindestens eine oder mehrere bevorzugt als alpha-1,4-verknüpfte D-Glucoseeinheiten vorliegen.

Besonders bevorzugt sind indes Steviolglykoside, die der folgenden Zusammensetzung folgen:
(a1) etwa 1 bis etwa 50, vorzugsweise etwa 1 bis 15 Gew.-% Rebaudioside A oder seine Isomere, wobei eines oder mehrere der Rebaudiosid A-Isomere in Bezug auf Rebaudiosid A zu 100:1 bis 1:100 vorliegen können,
(a2) etwa 0,1 bis etwa 30, vorzugsweise etwa 1 bis 15 Gew.-% Rebaudioside B oder seine Isomere, wobei eines oder mehrere der Rebaudiosid B-Isomere in Bezug auf Rebaudiosid B zu 100:1 bis 1:100 vorliegen können,
(a3) etwa 1 bis etwa 50, vorzugsweise etwa 1 bis etwa 15 Gew.-% Rebaudioside C oder seine Isomere, wobei eines oder mehrere der Rebaudiosid C-Isomere in Bezug auf Rebaudiosid C zu 100:1 bis 1:100 vorliegen können,
(a4) etwa 1 bis etwa 25, vorzugsweise etwa 1 bis etwa 15 Gew.-% Rebaudioside D oder seine Isomere, wobei eines oder mehrere der Rebaudiosid D-Isomere in Bezug auf Rebaudiosid D zu 100:1 bis 1:1000 vorliegen können,,
(a5) etwa 1 bis etwa 25, vorzugsweise etwa 1 bis etwa 20 Gew.-% Steviosid oder seine Isomere, wobei eines oder mehrere der Steviosid-Isomere in Bezug auf Steviosid zu 100:1 bis 1:100 vorliegen können,, und
(a6) etwa 1 bis etwa 5, vorzugsweise etwa 2 bis etwa 4 Gew.-% Steviolbiosid der seine Isomere, wobei eines oder mehrere der Steviolbiosid -Isomere in Bezug auf Steviolbiosid zu 100:1 bis 1:100 vorliegen können,,
(a7) optional auch 1 bis 50 Gew.-%, vorzugsweise 1 bis 10 % Rubusosid der seine Isomere, wobei eines oder mehrere der Rubusosid-Isomere in Bezug auf Rubusosid zu 100:1 bis 1:100 vorliegen können,
(a8) optional auch 1 bis 15 Gew.-% Rebaudiosid E der seine Isomere, wobei eines oder mehrere der Rebaudiosid E -Isomere in Bezug auf Rebaudiosid E zu 100:1 bis 1:100 vorliegen können,
(a9) optional auch 1 bis 15 Gew.-% Rebaudiosid F der seine Isomere, wobei eines oder mehrere der Rebaudiosid F -Isomere in Bezug auf Rebaudiosid F zu 100:1 bis 1:100 vorliegen können,
(a9) optional auch 1 bis 15 Gew.-% Dulcosid A der seine Isomere, wobei eines oder mehrere der Dulcosid A -Isomere in Bezug auf Dulcosid A zu 100:1 bis 1:100 vorliegen können,
(a10) und 1 bis 90, bevorzugt 20 bis 80 % höhere Steviolglycoside mit 5 bis zu 50, bevorzugt 6 bis 16 glycosidischen Einheiten enthalten sein können
wobei die Maßgabe besteht, dass sich die Mengenangaben zu 100 Gew.-% ergänzen.

Besonders vorteilhaft sind indes Steviolglykoside, die der vorhergehenden Zusammensetzung folgen und dabei
(a1) zwischen 1 und 5 Gew.-% Rebaudiosid A und Rebaudiosid A-Isomere, wobei eines oder mehrere der Rebaudiosid A-Isomere in Bezug auf Rebaudiosid A zu 5:1 bis 1:5 vorliegen,
(a2) zwischen 0.1 und 1 Gew.-% Rebaudiosid B und Rebaudiosid B-Isomere,
(a3) zwischen 1 und 5 Gew.-% Rebaudiosid C und Rebaudiosid C-Isomere,
(a4) zwischen 2 und 10 Gew.-% Rebaudiosid D und Rebaudiosid D-Isomere, wobei eines oder mehrere der Rebaudiosid D-Isomere in Bezug auf Rebaudiosid D größer 1:100 bis 1:500 vorliegen,
(a5) weniger als 5 Gew.-% Steviosid und Steviosid-Isomere, und
(a6) weniger als 5 Gew.-% Steviolbiosid und Steviolbiosid-Isomere enthalten sind.

Ganz besonders vorteilhaft sind indes Steviolglykoside, die der vorhergehenden Zusammensetzung folgen und dabei
(a1) weniger als 5 Gew.-% Rebaudiosid A,
(a2) weniger als 2 Gew.-% Rebaudiosid B,
(a3) weniger als 5 Gew.-% Rebaudiosid C,
(a4) weniger als 0.5 % Rebaudiosid D, und
(a5) weniger als 5 Gew.-% Steviosid,
enthalten.

Diese Steviolglykosidzusammensetzungen lassen sich nach den weiter oben beschriebenen enzymatischen oder fermentativen Verfahren der WO 2012 112180 A1, WO 2012 128775 A1, WO 2012 129451 A1 sowie WO 2013 096420 A1 aus einem oder mehreren der folgenden Stevioside erhalten: Rebaudiosid A, Rebaudiosid B, Rebaudiosid C, Rebaudiosid D, Rebaudiosid E, Rebaudiosid F, Steviosid, Steviolbiosid, Rubusosid, Dulcosid A.

Die genannten Stoffe Rebaudiosid A, Rebaudiosid B, Rebaudiosid C, Rebaudiosid D, Rebaudiosid E, Rebaudiosid F, Steviosid, Steviolbiosid, Rubusosid, Dulcosid A lassen sich in an sich bekannter Weise durch Extraktion der Blätter von *Stevia rebaudiana* oder *Rubus suavissimus* mit wässrigem Ethanol herstellen, wobei die Extrakte in der Regel - ohne Aufreinigung - etwa 1 Gew.-% Glykoside enthalten.

### STÄRKEABBAUPRODUKTE

Die Stärkeabbauprodukte, die die Komponente () bilden, haben in den Mischungen die Funktion von Trägerstoffen. Vorzugsweise handelt es sich dabei um Dextrine bzw. Maltodextrine, die von ihrer Molekülgröße her zwischen Oligosacchariden und Stärke liegen. Üblicherweise kommen sie in Form von weißem bzw. hellgelbem Pulver vor. Sie werden hauptsächlich aus Weizen-, Kartoffel- und Maisstärke durch trockene Erhitzung (>150 °C) oder unter Säureeinwirkung gewonnen. In der Natur wird Dextrin zum Beispiel von *Bacterium macerans* erzeugt. Dextrine entstehen auch durch den enzymatischen Abbau von Stärke durch Amylase.

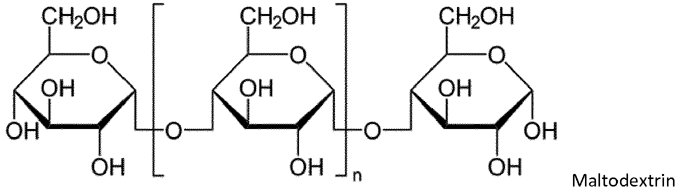

Maltodextrine, die in besonderer Weise geeignet sind, weisen etwa 3 bis etwa 20, vorzugsweise etwa 5 bis etwa 15 Dextrose-Äquivalente (DE) auf. Hierunter ist der prozentuale Anteil der reduzierenden Zucker in der Trockenmasse zu verstehen.

Die Komponenten (a) und (b) sind in den Stoffgemischen üblicherweise im Gewichtsverhältnis von etwa 60:40 bis etwa 90:10 und vorzugsweise etwa 70:30 bis 80:20 enthalten.

### PHENOLISCHE SÜSSVERSTÄRKENDE AROMASTOFFE

Phenolische süßverstärkende Aromastoffe, die die Gruppe (c) bilden, sind dadurch charakterisiert, dass sie aus derGruppe der Flavonoidabkömmlinge, insbesondere der Flavanone, der Dihydrochalkone und der Neoisoflavanone abgeleitet sind mit der Maßgabe, dass mindestens eine p-Hydroxyphenyl- oder eine 4-Hydroxy-3-methoxyphenyl- oder eine 4-Methoxy-3-hydroxyphenylgruppe enthalten ist und bevorzugt ausgewählt werden aus Phloretin, 1-(2,4-Dihydroxy-phenyl)-3-(3-hydroxy-4-methoxy-phenyl)-propan-1-on, 7,3-Dihydroxy-4'methoxy-flavan oder 5-Hydroxy-4-(4-hydroxy-3-methoxy-phenyl)-7-methoxy-2-chromanon und vorzugsweise Hesperetin.

Hesperetin ist das Aglycon des Hesperidins und gehört zur Gruppe der Flavanone:

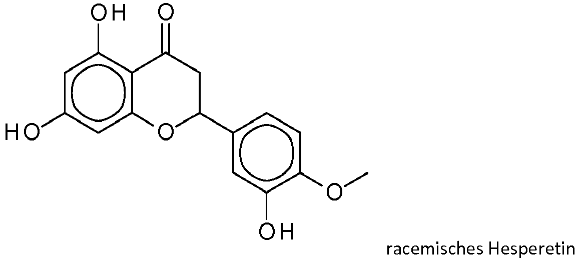

Hesperetin kann sowohl als (S)- als auch als (R)-Form als auch in beliebiger Mischung der beiden Formen sowie in Form physiologisch verträglicher Salze verwendet werden; dabei wird auf die Schrift WO 2007 014,879 sowie deren Äquivalente verwiesen.

Phloretin ist das Aglycon des Phloridizins und gehört zur Gruppe der Dihydrochalkone:

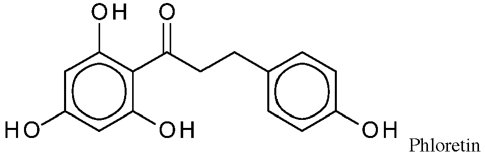

Dabei wird bezüglich der Zubereitung auf die Schrift EP 1,998,636 sowie deren Äquivalente verwiesen.

Ebenso gehört das 1-(2,4-Dihydroxy-phenyl)-3-(3-hydroxy-4-methoxy-phenyl)-propan-1-on zur Gruppe der Dihydrochalkone:

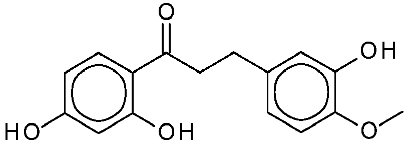

Dabei wird bezüglich der Zubereitung auf die Schrift EP 2,353,403 sowie deren Äquivalente verwiesen.

7,3-Dihydroxy-4'methoxyflavan gehört zur Gruppe der Flavane:

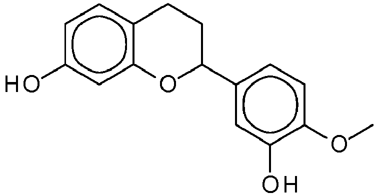

Dabei wird bezüglich der Zubereitung auf die Schrift EP 2,253,226 sowie deren Äquivalente verwiesen.

Das Neoisoflavonoid 5-Hydroxy-4-(4-hydroxy-3-methoxy-phenyl)-7-methoxy-2-chromanon ist in der folgenden Abbildung aufgeführt und wurde in EP 2,570,036 offenbart:

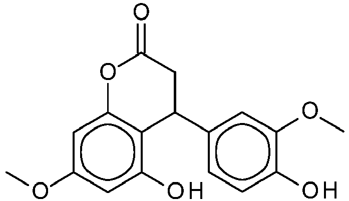

### STOFFGEMISCHE

Vorzugsweise zeichnen sich die erfindungsgemäßen Stoffgemische dadurch aus, dass sie die Komponenten (a+b) und (c) im Gewichtsverhältnis vorzugsweise etwa 5:95 bis etwa 75:25, besonders bevorzugt etwa 25:75 bis 75:25 und ganz besonders bevorzugt etwa 5:95 bis 25:75 enthalten. Eine typische Zusammensetzung der Mischungen sieht daher beispielsweise wie folgt aus:
(a) etwa 1 bis etwa 99 Gew.-%, vorzugsweise 5 bis 50 Gew.-% Steviolglykoside, besonders bevorzugt
(b) etwa 0,1 bis etwa 15 Gew.-%, vorzugsweise etwa 0,5 bis etwa 10 Gew.-% Maltodextrine und
(c) etwa 0,1 bis etwa 20Gew.-%, vorzugsweise etwa 0,5 bis 10 Gew.-% eines oder mehrerer der phenolischen süßverstärkenden Aromastoffe
mit der Maßgabe, dass sich die Mengenangaben zu 100 Gew.-% ergänzen.

### ORALE ZUBEREITUNGEN

Ein weiterer Gegenstand der vorliegenden Erfindung betrifft Zubereitungen zur oralen Aufnahme, die die erfindungsgemäßen Mischungen der Komponenten (a), (b) und (c) vorzugsweise in Mengen von 0,00001 bis etwa 2 Gew.-% enthalten. Weitere bevorzugte Zusatzmengen sind 0,0001 bis etwa 1,5 Gew.-%, insbesondere 0,001 bis etwa 1 Gew.-%, weiter bevorzugt etwa 0,01 bis etwa 0,5 Gew.-% und ganz besonders bevorzugt etwa 0,05 bis 0,1 Gew.-%

### NAHRUNGSMITTEL

In einer ersten Ausführungsform können die oralen Zubereitungen ausgewählt sein aus der Gruppe, die gebildet wird von
- Backwaren, beispielsweise Brot, Trockenkekse, Kuchen, sonstiges Gebäck,
- Süßwaren (beispielsweise Schokoladen, Schokoladenriegelprodukte, sonstige Riegelprodukte, Fruchtgummi, Hart- und Weichkaramellen, Kaugummi),
- alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte,
- Frucht- oder Gemüsesaftzubereitungen,
- Instantgetränke (beispielsweise Instant-Kakao-Getränke, Instant-Tee-Getränke, Instant-Kaffeegetränke, Instant-Fruchtgetränke),
- Fleischprodukte (beispielsweise Schinken, Frischwurst- oder Rohwurstzubereitungen, gewürzte oder marinierte Frisch- oder Pökelfleischprodukte),
- Eier oder Eiprodukte (Trockenei, Eiweiß, Eigelb), Getreideprodukte (beispielsweise Frühstückscerealien, Müsliriegel, vorgegarte Fertigreis-Produkte),
- Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte),
- Produkte aus Sojaprotein oder anderen Sojabohnen-Fraktionen (beispielsweise Sojamilch und daraus gefertigte Produkte, Fruchtgetränke mit Sojaprotein, Sojalecithinhaltige Zubereitungen, fermentierte Produkte wie Tofu oder Tempe oder daraus gefertigte Produkte),
- Produkte aus anderen pflanzlichen Proteinquellen, beispielsweise Haferprotein-Getränke,
- Fruchtzubereitungen (beispielsweise Konfitüren, Fruchteis, Fruchtsoßen, Fruchtfüllungen),
- Gemüsezubereitungen (beispielsweise Ketchup, Soßen, Trockengemüse, Tiefkühlgemüse, vorgegarte Gemüse, eingekochte Gemüse),
- Knabberartikel (beispielsweise gebackene oder frittierte Kartoffelchips oder Kartoffelteigprodukte, Extrudate auf Mais- oder Erdnussbasis),
- Produkte auf Fett- und Ölbasis oder Emulsionen derselben (beispielsweise Mayonnaise, Remoulade, Dressings),
- sonstige Fertiggerichte und Suppen (beispielsweise Trockensuppen, Instant-Suppen, vorgegarte Suppen),
- Gewürze, Würzmischungen sowie insbesondere Aufstreuwürzungen (englisch: Seasonings), die beispielsweise im Snackbereich Anwendung finden.

Besonders bevorzugt sind hier Süßwaren, Milchprodukte und ganz besonders nicht-alkoholische Getränke, wobei gesüßte Getränke bevorzugt sind.

### HILFS- UND ZUSATZSTOFFE

Diese Nahrungsmittel können typischerweise weitere Hilfs- und Zusatzstoffe enthalten, darunter insbesondere Süßstoffe, Lebensmittelsäuren, Säureregulatoren, Verdickungsmittel und insbesondere weitere Aromastoffe.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
synthetische, d.h. in der Regel enzymatisch hergestellte Stärke oder Zuckerhydrolysate (Invertzucker, Fructosesirup);
Fruchtkonzentrate (z.B. auf Basis von Äpfeln oder Birnen);
Zuckeralkohole (z.B. Erythritol, Threitol, Arabitol, Ribotol, Xylitol, Sorbitol, Mannitol, Dulcitol, Lactitol);
Proteine (z.B. Miraculin, Monellin, Thaumatin, Curculin, Brazzein);
Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
Süß schmeckende Aminosäuren (z.B. Glycin, D-Leucin, D-Threonin, D-Asparagin, D-Phenylalanin, D-Tryptophan, L-Prolin);
Weitere süß schmeckende niedermolekulare Substanzen, wie z.B. Hernandulcin, Dihydrochalconglykoside, insbesondere Neohesperidindihydrochalkon und Naringinchalkon, Glycyrrhizin, Glycerrhetinsäure, ihre Derivate und Salze, Extrakte von Lakritz (Glycyrrhizza glabra ssp.), *Lippia dulcis* Extrakte, *Momordica* ssp. Extrakte oder
Einzelsubstanzen wie z.B. Momordica grosvenori [Luo Han Guo] und die daraus gewonnenen Mogroside, *Hydrangea dulcis* oder Extrakte oder Phyllodulcin.
Balansine aus *Mycetia balansae.*

### Lebensmittelsäuren

Die Nahrungsmittel können Carbonsäuren enthalten. Säuren im Sinne der Erfindung sind bevorzugt in Lebensmitteln zulässige Säuren, insbesondere die hier genannten:

| | |
|---|---|
| E 260 | - Essigsäure |
| E 270 | - Milchsäure |
| E 290 | - Kohlendioxid |
| E 296 | - Apfelsäure |
| E 297 | - Fumarsäure |
| E 330 | - Citronensäure |
| E 331 | - Natriumcitrat |
| E 332 | - Kaliumcitrat |
| E 333 | - Calciumcitrat |
| E 334 | - Weinsäure |
| E 335 | - Natriumtartrat |
| E 336 | - Kaliumtartrat |
| E 337 | - Natrium-Kaliumtartrat |
| E 338 | - Phosphorsäure |
| E 353 | - Metaweinsäure |
| E 354 | - Calciumtartrat |
| E 355 | - Adipinsäure |
| E 363 | - Bernsteinsäure |
| E 380 | - Triammoniumcitrat |
| E 513 | - Schwefelsäure |
| E 574 | - Gluconsäure |
| E 575 | - Glucono-delta-Lacton |

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:

| | |
|---|---|
| E 170 | - Calciumcarbonat |
| E 260-263 | - Essigsäure und Acetate |
| E 270 | - Milchsäure |
| E 296 | - Äpfelsäure |
| E 297 | - Fumarsäure |
| E 325-327 | - Lactate (Milchsäure) |
| E 330-333 | - Citronensäure und Citrate |
| E 334-337 | - Weinsäure und Tartrate |
| E 339-341 | - Orthophosphate |
| E 350-352 | - Malate (Äpfelsäure) |
| E 450-452 | - Di-, Tri- und Polyphosphate |
| E 500-504 | - Carbonate (Kohlensäure) |
| E 507 | - Salzsäure und ChlorideE 513-517Schwefelsäure und Sulfate |
| E 524-528 | - Hydroxide |
| E 529-530 | - Oxide |
| E 355-357 | - Adipinsäure und Adipate |
| E 574-578 | - Gluconsäure und Gluconate |

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:

| | |
|---|---|
| E 400 | - Alginsäure |
| E 401 | - Natriumalginat |
| E 402 | - Kaliumalginat |
| E 403 | - Ammoniumalginat |
| E 404 | - Calciumalginat |
| E 405 | - Propylenglycolalginat |
| E 406 | - Agar Agar |
| E 407 | - Carrgeen, Furcelleran |
| E 407 | - Johannisbrotkernmehl |
| E 412 | - Guarkernmehl |
| E 413 | - Traganth |
| E 414 | - Gummi arabicum |
| E 415 | - Xanthan |
| E 416 | - Karaya (Indischer Traganth) |
| E 417 | - Tarakernmehl (Peruanisches Johannisbrotkernmehl) |
| E 418 | - Gellan |
| E 440 | - Pektin, Opekta |
| E 440ii | - Amidiertes Pektin |
| E 460 | - Mikrokristalline Cellulose, Cellulosepulver |
| E 461 | - Methylcellulose |
| E 462 | - Ethylcellulose |
| E 463 | - Hydroxypropylcellulose |
| E 465 | - Methylethylcellulose |
| E 466 | - Carboxymethylcellulose, Natriumcarboxymethylcellulose |

### Aromastoffe

Die erfindungsgemäßen oralen Zubereitungen können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### MUND- UNDZAHNPFLEGEMITTEL

In einer weiteren Ausführungsform können die oralen Zubereitungen ausgewählt sein aus der Gruppe, die gebildet wird von Mund- und Zahnpflegemitteln zu denen auch Mundwässer und Kaugummis gezählt werden.

Konkrete Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### KAUGUMMIS

Soweit es sich bei den oralen Zubereitungen um Kaugummis handelt, enthalten diese typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500,

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### GEWERBLICHE ANWENDBARKEIT

Weitere Gegenstände der vorliegenden Erfindung sind zum einen auf ein Verfahren zur geschmacklichen Optimierung von Zubereitungen für die orale Aufnahme gerichtet, das sich dadurch auszeichnet, dass man diesen etwa 0,0001 bis etwa 2 Gew.-% der Mischungen enthaltend die Komponenten (a), (b) und (c) zusetzt. Ebenfalls umfasst wird durch die Erfindung ein Verfahren zur Löslichkeitsverbesserung von Aromastoffen in Zubereitungen zur oralen Aufnahme, welches sich dadurch auszeichnet, dass man diese zusammen mit 0,0001 bis 2 Gew.-% der Mischungen enthaltend die Komponenten (a), (b) und (c) einsetzt.

Schließlich betrifft die Erfindung zum einen die Verwendung der Mischungen enthaltend die Komponenten (a), (b) und (c) zum einen als Aromastoffe für Zubereitungen für die orale Aufnahme sowie zum anderen als Löslichkeitsverbesserer für andere Aromastoffe.

Bezüglich der bevorzugten Zusammensetzungen der Mischungen, den bevorzugten Einsatz- bzw. Zusatzmengen sowie der Natur der Aromastoffe wird auf die vorstehenden Ausführungen verwiesen.

### BEISPIELE

**Tabelle 1**

| Zusammensetzung der Steviolglycosid-Mischungen, enthaltend Stärkeabbauprodukte | | | | | |
|---|---|---|---|---|---|
| **Inhaltsstoffe** | **A** | **B** | **C** | **D** | **E** |
| Stärkeabbauprodukte (Maltodextrine) | 3,4 | 4,8 | 4,9 | 15,0 | 15,0 |
| Rebaudiosid A | 0,9 | 1,0 | 0,8 | 3,6 | 1,4 |
| Rebaudiosid B | 1,3 | 1,0 | 0,5 | 0,51 | 0,48 |
| Rebaudiosid C | 2,6 | 2,0 | 1,0 | 0,91 | 1,2 |
| Rebaudiosid D | 0,1 | 0,2 | 0,2 | 0,082 | 0,082 |
| Steviosid | 3,0 | 3,1 | 3,7 | 1,5 | 3,2 |
| Steviolbiosid | 1,2 | 1,6 | 2,0 | 0,13 | 0,64 |
| Steviolglycoside (3fach, Steviosid-Isomere, Dulcosid A-Isomere, Rebaudiosid B-Isomere) | 11,6 | 9,8 | 12,1 | 10,0 | 10,0 |
| Steviolglycoside (4fach, Rebaudiosid A-Isomere, Rebaudiosid C-Isomere, Rebaudiosid E-Isomere, Rebaudiosid F-Isomere) | 10,5 | 10,4 | 7,1 | 2,6 | 5,3 |
| Steviolglycoside (5fach, Rebaudiosid D-Isomere) | 8,7 | 8,5 | 8,4 | 5,72 | 4,44 |
| Steviolglycoside (6fach) | 10,3 | 9,8 | 8,4 | 8 | 8 |
| Steviolglycoside (7fach) | 10,7 | 10,3 | 12,6 | 13 | 13 |
| Steviolglycoside (8fach) | 6,2 | 6,9 | 6,8 | 7,0 | 7,0 |
| Steviolglycoside (9fach) | 7,0 | 6,3 | 7,2 | 7,0 | 7,0 |
| Steviolglycoside (10fach) | 4,3 | 4,8 | 4,8 | 5,0 | 5,0 |
| Steviolglycoside (11fach) | 2,7 | 3,9 | 4,6 | 4,0 | 4,0 |
| Steviolglycoside (12fach) | 2,7 | 2,5 | 2.1 | 2,0 | 2,0 |
| Steviolglycoside (13fach) | 1,1 | 1,3 | 0,4 | 0,5 | 0,5 |
| Steviolglycoside (14fach) | 0,6 | 0,4 | 0,6 | 1 | 1 |
| Steviolglycoside (15fach) | 0,6 | 0,4 | 0,6 | 0,4 | 0,4 |
| Steviolglycoside (16+ fach) | 2,0 | 2,3 | 2,1 | 2,1 | 2,1 |
| Wasser (Karl-Fischer) | 7,3 | 6,1 | 5,2 | 5 | 6 |
| **TOTAL** | **98,7** | **97,5** | **96,0** | **95,1** | **97,7** |

**Tabelle 2**

| Zusammensetzung von Steviolglycosid-Mischungen | | | | | | | | | | | |
|---|---|---|---|---|---|---|---|---|---|---|---|
| **Inhaltstoffe** | **A** | **B** | **C** | **D** | **E** | **F** | **G** | **H** | **I** | **J** | **K** |
| Steviolbiglucoside (z.B. Steviolbiosid, Rubusosid) | 1,1 | 0,6 | 1,3 | 0,6 | 1 | 2,4 | 7,2 | 6,7 | 1,3 | 10,6 | 0,8 |
| Stevioltriglucoside (z.B. Steviosid, Rebaudiosid B und Isomere) | 2,9 | 7,7 | 7,5 | 3,4 | 2,4 | 5,7 | 9,3 | 8,7 | 12 | 5,7 | 1,6 |
| Stevioltetraglucooside (e.g. Reb A, C und Isomere) | 10,3 | 12,7 | 13,1 | 9,7 | 8,5 | 6,6 | 19,8 | 20,8 | 24,3 | 13,6 | 8,3 |
| Steviolpentaglucooside (e.g. Reb D und Isomere) | 13,3 | 19,2 | 20,3 | 10,4 | 7,5 | 9 | 16,5 | 16,3 | 26 | 15,5 | 10,7 |
| Steviolhexaglucoside | 20,2 | 10,4 | 10,8 | 12,6 | 14 | 12,9 | 18,9 | 20,6 | 4,7 | 16,7 | 9 |
| Steviolheptaglucoside | 14,4 | 9,7 | 8 | 10,6 | 10,7 | 12,1 | 11,8 | 11,4 | 6,8 | 13,6 | 13 |
| Stevioloctaglucoside | 12 | 9,5 | 8,9 | 10,6 | 9,5 | 10,8 | 6,2 | 7,4 | 9,7 | 7,9 | 11,5 |
| Steviolnonaglucoside | 9,9 | 6,9 | 8,7 | 9,6 | 9 | 8,6 | 3,9 | 2,3 | 4,2 | 6,5 | 11,8 |
| Stevioldecaglucoside | 6,1 | 6,4 | 5,1 | 6,9 | 11,4 | 6,8 | 1,5 | 1,8 | 2,8 | 4,7 | 6,7 |
| Steviolundecaglucoside | 3,1 | 4,5 | 4,3 | 6,5 | 8,4 | 7 | 1,4 | 0,5 | 1,5 | 1,5 | 9,7 |
| Stevioldodecaglucoside | 1,8 | 5,3 | 4,8 | 2,8 | 4,1 | 4,6 | 0,3 | 0,3 | 1,2 | 0,3 | 4,1 |
| Stevioltridecaglucoside | 0,5 | 2,1 | 2 | 5,4 | 5,4 | 5,5 | 0,2 | 0,2 | 2,8 | 0,3 | 4,5 |
| Stevioltetradecaglucoside | 0,4 | 0,3 | 0,6 | 2,8 | 1,4 | 1,2 | 0,2 | 0,2 | 0,9 | 0,3 | 3,4 |
| Steviolpentadecaglucoside | 0,3 | 0,3 | 0,3 | 2,3 | 1,8 | 0,5 | 0,2 | 0,2 | 0,1 | 0,2 | 1,5 |
| | | | | | | | | | | | |
| Steviol-16-22-glucoside | 1,3 | 1,5 | 1,4 | 2,5 | 1,7 | 2,1 | 0,7 | 0,7 | 0,6 | 0,9 | 1,1 |
| Steviol-22-28-glucoside | 0,6 | 0,7 | 0,6 | 0,6 | 0,8 | 0,9 | 0,4 | 0,4 | 0,4 | 0,4 | 0,4 |
| Steviol-28-60-glucosides | 1,1 | 1,3 | 1,3 | 1,5 | 1,4 | 2 | 1 | 1 | 0,5 | 0,8 | 0,9 |
| **Summe Stevioside** | **99,3** | **99,1** | **99,0** | **98,9** | **99,0** | **98,7** | **99,5** | **99,5** | **99,8** | **99,5** | **99,0** |

**Tabelle 3**

| Aromazubereitungen | | | | | | | | | | |
|---|---|---|---|---|---|---|---|---|---|---|
| **Zusammensetzung** | **A** | **B** | **C** | **D** | **E** | **F** | **G** | **H** | **I** | **J** |
| Steviolglycosidmischung A enthaltend Maltodextrine aus Beispiel 1a | 10 | 20 | 10 | | | 10 | | 10 | | |
| Steviolglycosidmischung E enthaltend Maltodextrine aus Beispiel 1a | | | | 20 | 15 | | 7,5 | | | |
| Steviolglycosidmischung F Beispiel 1b | | | | | | | | | 20 | |
| Steviolglycosidmischung I Beispiel 1b | | | | | | | | | | 20 |
| Hesperetin aus Citrus (racemisch, >80 %) | 2 | | | | | | | | 2 | |
| (S)-Hesperetin aus Honigbusch (> 95%) | | | 1 | | | | 1,5 | | | |
| Phloretin | | | 2 | | | | 2 | | | |
| Hesperetin | | 3 | | 3 | 1 | | | 2 | | 3 |
| 1-(2,4-Dihydroxy-phenyl)-3-(3-hydroxy-4-methoxyphenyl)-propan-1-on | | | | | | 2 | | | | |
| 7,3-Dihydroxy-4'methoxyflavan | | | | | 2 | | | | | |
| 5-Hydroxy-4-(4-hydroxy-3-methoxy-phenyl)-7-methoxy-2- chromanon | | | | | | 2 | | | | |
| Glycerin | 10 | 20 | | 30 | | 20 | 15 | 30 | 10 | 20 |
| Gummi arabicum-Lösung (20%) | | | | | | | | 10 | | |
| 1,2-Propylenglycol | Ad 100 | | | | | | | | | |

### BEISPIEL 1

### Getränkeformulierung

Verschiedene Stoffgemische auf Basis von Steviolglykosiden wurden zur Herstellung von einfachen Softdrinks verwendet und die Produkte 48 h bei 20°C gelagert. Anschließend wurden die geschmacklichen Eigenschaften (Deskriptoren: Anfangssüße, Süßintensität, Zuckergeschmack/Mundfülle) von einem Panel bestehend aus 8 geschulten Testern auf einer Linien-Skala von 0 (nicht vorhanden) bis 10 (stark ausgeprägt) bewertet. Die Zusammensetzungen und Ergebnisse sind in der nachfolgenden **Tabelle 4** zusammengefasst. Die Ausführungen 1 bis 2 sind erfindungsgemäß, die Beispiele V1 bis V3 dienen zum Vergleich. Das Beispiel C entspricht dem Standard, d.h. der geschmacklichen Beurteilung des Produktes ohne Zusatz von Aroma.

**Tabelle 4**

| Geschmackliche Eigenschaften von Softdrinkformulierungen | | | | | | |
|---|---|---|---|---|---|---|
| **Zusammensetzung** | **C** | **1** | **2** | **V1** | **V2** | **V3** |
| Sucrose | 10 | 7 | 7 | 7 | 7 | 7 |
| Zitronensäure | 0.2 | 0.2 | 0.2 | 0.2 | 0.2 | 0.2 |
| Steviolglycosidmischung A enthaltend Maltodextrine aus Beispiel 1 | - | 0.0045 | 0.0045 | - | 0.0045 | - |
| Hesperetin | - | 0.00075 | 0.0012 | - | - | 0.00075 |
| Zitronenaroma | 0,1 | 0,1 | 0,1 | 0,1 | 0,1 | 0,1 |
| Wasser | | | | Ad 100 | | |

| ***Geschmackliche Beurteilung*** | | | | | | |
|---|---|---|---|---|---|---|
| Anfangssüße | 5,7 | 5,2 | 5,2 | 3,2 | 4,2 | 3,2 |
| Süßintensität | 6,5 | 5,7 | 5,8 | 3,5 | 4,71 | 3,8 |
| Zuckergeschmack und Mundfülle | 5,8 | 4,7 | 5,4 | 3,0 | 4,6 | 3,3 |
| Bitterkeit beim Nachgeschmack | 0,2 | 0,5 | 0,6 | 0,2 | 0,7 | 0,4 |

Eindeutig ist die hochzuckerhaltige Kontrollprobe C am besten bewertet worden, V1 am schlechtesten. Bei Zusatz geringen Mengen Hesperetin alleine (V3) konnten alle positiven Geschmackswerte nicht weiter verbessert werden. Die Steviolglycoside alleine (V2) führten zur Verbesserung des Süßeindrucks, allerdings auch zur verstärkten Bitterkeit im Nachgeschmack. Bei den erfindungsgemäßen Ausführungen 1 und 2 konnten die Anfangssüße, die Süßintensität und der Zuckergeschmack/Mundfülle wieder weitgehend vergleichbar zur Vollzuckervariante C hergestellt werden und die Bitterkeit im Vergleich zu reinen Steviolglycosiden (V2) dabei deutlich gesenkt werden.

### BEISPIEL 2

### Stabilität von Getränken

Wiederum wurden verschiedene Stoffgemische auf Basis von Steviolglykosiden zur Herstellung von Softdrinks verwendet. Die Produkte wurden 48 h bei 20°C gelagert und die Löslichkeit der Aromastoffe in Abhängigkeit der Konzentration der erfindungsgemäßen Gemische optisch auf einer Skala (0) = vollständig gelöst, (1) = einzelne Ausscheidungen/Trübungen und (2) = deutliche Abscheidungen/Partikelbildung beurteilt. Die Ergebnisse sind in **Tabelle 5** zusammengefasst.

**Tabelle 5**

| Löslichkeitsuntersuchungen | | | | |
|---|---|---|---|---|
| **Zusammensetzung** | **3** | **4** | **V4** | **V5** |
| Sucrose | 7 | 7 | 7 | 7 |
| Zitronensäure | 0.2 | 0.2 | 0.2 | 0.2 |
| Steviolglycosidmischung A enthaltend Maltodextrine aus Beispiel 1 | 0.0045 | 0.0045 | - | 0.0045 |
| Hesperetin | 0.00075 | 0.0012 | - | - |
| Zitronenaroma | 0,1 | 0,1 | 0,1 | 0,1 |
| Wasser | Ad 100 | | | |

| ***Löslichkeitsbe urteilung*** | | | | |
|---|---|---|---|---|
| Nach 12 h | 0 | 0 | 1 | 0 |
| Nach 24 h | 0 | 0 | 2 | 0 |
| Nach 48 h | 0 | 1 | 2 | 0 |

Die Ausführungen 3 und 4 zeigen im Vergleich zu den Vergleichsbeispielen V4 und V5 als erstes, dass der Zusatz von der Steviolglykosid/Maltodextrin-Mischung die Löslichkeit des Hesperetins deutlich verbessert. Auch im Hinblick auf die verschiedenen zusätzlichen Aromastoffe wirkt sich der Zusatz der erfindungsgemäßen Mischungen löslichkeitsverbessernd aus.

Im Folgenden wird die Erfindung an Hand weiterer Formulierungsbeispiele illustriert.

**Tabelle 6**

| Erfrischungsgetränke (Mengenangaben als Gew.-%) | | | | | | | |
|---|---|---|---|---|---|---|---|
| **Inhaltsstoffe** | **A** | **B** | **C** | **D** | **E** | **F** | **G** |
| Sucrose | 10 | 10 | 7 | - | - | 8 | 7 |
| Glucose/Fructose Sirup | - | - | - | - | 10 | - | - |
| Steviolglycosidmischung A enthaltend Maltodextrine aus Beispiel 1 | 0,0045 | 0,005 | 0,003 | 0,01 | 0,0045 | 0,0025 | 0,001 |
| Zitronensäure | 0,15 | 0,15 | 0,06 | 0,15 | 0,15 | 0,15 | 0,15 |
| Phosphorsäure | - | - | 0,07 | - | - | - | - |
| Zuckercoleur | - | - | 0,14 | - | - | - | - |
| Koffein | - | - | 0,01 | - | - | - | - |
| Zitrusaroma | 0,1 | 0,05 | - | 0,1 | 0,1 | 0,1 | 0,1 |
| Limonenaroma | - | 0,05 | - | - | - | - | - |
| Getränkeemulsion Typ "Cola" | - | - | 0,05 | - | - | - | - |
| Phloretin | - | - | 0,001 | 0,0005 | - | 0,0005 | 0,01 |
| Hesperetin | 0,00075 | 0,0012 | 0,00075 | 0,0012 | 0,00075 | 0,00075 | 0,01 |
| Homoeriodictyol-Na | - | - | 0,005 | 0,005 | - | - | - |
| Wasser | Ad 100 | | | | | | |

Die Zutaten wurden in der angegebenen Reihenfolge gemischt und mit Wasser auf 100 % aufgefüllt. Die Mischungen werden in Glasflaschen gefüllt und carbonisiert.

**Tabelle 7**

| Hartkaramellen (Mengenangaben als Gew.-%) | | | | |
|---|---|---|---|---|
| **Inhaltsstoffe** | **A** | **B** | **C** | **D** |
| Zucker | 74,50 | | | |
| Palatinit, Type M | - | 74,00 | 75,50 | 75,00 |
| Zitronensäure | 0,5 | 1,0 | 0,5 | - |
| Farbstoff, gelb | - | 0,01 | - | - |
| Farbstoff, rot | - | - | 0,01 | - |
| Farbstoff, blau | 0,01 | - | - | 0,01 |
| Pfefferminzaroma | 0,1 | - | - | 0,1 |
| Zitrusaroma | - | 0,1 | - | - |
| Rote Beeren-Aroma | - | - | 0,1 | - |
| SWETA¹⁾ | 0,005 | 0,0045 | 0,006 | 0,003 |
| Balansin A | - | 0,005 | 0,010 | 0,005 |
| Hesperetin | 0,00075 | 0,001 | 0,0005 | 0,0004 |
| Phloretin | - | 0,002 | - | - |
| Wasser | ad 100 | ad 100 | ad 100 | ad 100 |

| | | | | |
|---|---|---|---|---|
| ¹⁾ Steviolglykosid/Maltodextrin-Mischung (80:20) der Firma Pure Circle | | | | |

**Tabelle 8**

| Joghurt mit niedrigem Fettgehalt (Mengenangaben als Gew.-%) | | | | |
|---|---|---|---|---|
| **Inhaltsstoffe** | **A** | **B** | **C** | **D** |
| Sucrose | 10 | 8 | 6 | - |
| Sucralose | - | 0,02 | - | 0,2 |
| Rebaudiosid A > 95 % | - | - | 0,025 | - |
| Saccharin | - | - | - | 0,3 |
| SWETA¹⁾ | 0,005 | 0,0045 | 0,01 | 0,050 |
| Sauerkirsch-Extrakt nach Beispiel 1 | 0,2 | 0,1 | 0,2 | 0,2 |
| Hesperetin | 0,00075 | 0,001 | 0,003 | 0,002 |
| Phloretin | - | - | 0,002 | 0,002 |
| Homoeriodictyol-Natriumsalz | - | - | - | 0,005 |
| Joghurt, 0,1 % Fett | zu 100 % auffüllen | | | |

**Tabelle 9**

| Fruchtgummis (Mengenangaben als Gew.-%) | | |
|---|---|---|
| **Inhaltsstoffe** | **A** | **B** |
| Saccharose | 34,50 | 8,20 |
| Glucosesirup, DE 40 | 31,89 | 30,09 |
| SWETA¹⁾ | 0,005 | 0,0045 |
| Iso Sirup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Farbstoff | 0,01 | 0,01 |
| Zitrusaroma | 0,20 | - |
| Kirscharoma | - | 0,10 |
| Hesperetin | 0,0075 | 0,0015 |
| Wasser | ad 100 | ad 100 |

**Tabelle 10**

| Zuckerfreies Kaugummi (Mengenangaben als Gew.-%) | |
|---|---|
| **Inhaltsstoffe** | **Gehalt** |
| Kaugummibasis | 30.00 |
| Sorbitolpulver | Ad 100 |
| SWETA¹⁾ | 0,05 |
| Palatinit | 9.50 |
| Xylitol | 2.00 |
| Mannitol | 3.00 |
| Aspartam | 0.10 |
| Acesulfam K | 0.10 |
| Emulgum / Emulgator | 0.30 |
| Sorbitol 70 %. in Wasser | 14.00 |
| Glycerin | 1.00 |
| Pfefferminzaroma | 1.50 |
| Hesperetin | 0,01 |

**Tabelle 11**

| Pudding (Mengenangaben in g) | | | |
|---|---|---|---|
| Inhaltsstoffe | A | B | C |
| Maisstärke | 38 | 38 | 38 |
| Zucker | 38 | 30 | 22,8 |
| Steviolglykoside | - | 0,05 | 0,08 |
| Hesperetin | - | 0,01 | 0,03 |
| Vanille-Aroma (Symrise) | 0,2 | 0,2 | 0,2 |
| Chinolin gelb | 0,02 | 0,02 | 0,02 |
| Milch | 500ml | 500ml | 500ml |

## Patentansprüche

1. Stoffgemische, enthaltend
(a) Steviolglykoside,
(b) Stärkeabbauprodukte und
(c) einen oder mehrere phenolische süßverstärkenden Aromastoffe, ausgewählt aus der Gruppe, die gebildet wird von Hesperetin, Phloretin, 1-(2,4-Dihydroxyphenyl)-3-(3-hydroxy-4-methoxy-phenyl)-propan-1-on, 7,3-Dihydroxy-4'-methoxy-flavan und 5-Hydroxy-4-(4-hydroxy-3-methoxy-phenyl)-7-methoxy-2-chromanon
wobei die Stoffgemische die Komponenten (a+b) und (c) im Gewichtsverhältnis 1:99 bis 99:1 enthalten.

2. Stoffgemische nach Anspruch 1, **dadurch gekennzeichnet, dass** die Steviolglykoside 2 bis 6 Glucoseeinheiten aufweisen.

3. Stoffgemische nach den Ansprüchen 1 und/oder 2, **dadurch gekennzeichnet, dass** die Steviolglykoside
(a1) 30 bis 50 Gew.-% Rebaudioside A,
(a2) 5 bis 10 Gew.-% Rebaudioside B,
(a3) 5 bis 10 Gew.-% Rebaudioside C,
(a4) 20 bis 30 Gew.-% Rebaudioside D,
(a5) 15 bis 25 Gew.-% Stevioside und
(a6) 1 bis 5 Gew.-% Steviolbioside
mit der Maßgabe enthalten, dass sich die Mengenangaben zu 100 Gew.-% ergänzen.

4. Stoffgemische nach mindestens einem der Ansprüche 1 bis 3, **dadurch gekennzeichnet, dass** die Stärkeabbauprodukte Dextrine darstellen.

5. Stoffgemische nach mindestens einem der Ansprüche 1 bis 4, **dadurch gekennzeichnet, dass** sie die Komponenten (a) und (b) im Gewichtsverhältnis 60:40 bis 90:10 enthalten.

6. Zubereitungen zur oralen Aufnahme, enthaltend die Mischungen nach Anspruch 1.

7. Zubereitungen nach Anspruch 6, **dadurch gekennzeichnet, dass** sie 0,00001 bis 2 Gew.-% der Mischung nach Anspruch 1 enthalten.

8. Zubereitungen nach den Ansprüchen 6 und/oder 7, **dadurch gekennzeichnet, dass** diese ausgewählt sind aus der Gruppe, die gebildet wird von Backwaren, Süßwaren, alkoholischen oder nicht-alkoholischen Getränken, Frucht- oder Gemüsesaftzubereitungen, Instantgetränke, Fleischprodukte, Eier oder Eiprodukte, Getreideprodukte, Milchprodukte, Produkte aus Sojaprotein oder anderen Sojabohnen-Fraktionen, Produkte aus anderen pflanzlichen Proteinquellen, Gemüsezubereitungen, Knabberartikel, Produkte auf Fett- und Ölbasis oder Emulsionen derselben, sonstige Fertiggerichte und Suppen, Gewürze, Würzmischungen sowie insbesondere Aufstreuwürzungen.

9. Zubereitungen nach den Ansprüchen 6 und/oder 7, **dadurch gekennzeichnet, dass** diese ausgewählt sind aus der Gruppe, die gebildet wird von Mund- und Zahnpflegemitteln, Mundwässern und Kaugummis.

10. Zubereitungen nach mindestens einem der Ansprüche 6 bis 9, **dadurch gekennzeichnet, dass** sie weiterhin Süßstoffe, Lebensmittelsäuren, Säureregulatoren, Verdickungsmittel und zusätzliche Aromastoffe enthalten.

11. Verfahren zur geschmacklichen Optimierung von Zubereitungen für die orale Aufnahme und zur Löslichkeitsverbesserung von Aromastoffen in Zubereitungen zur oralen Aufnahme, **dadurch gekennzeichnet, dass** man diesen 0,0001 bis 2 Gew.-% der Mischungen nach Anspruch 1 zusetzt.

12. Verwendung der Mischungen nach Anspruch 1 als Aromastoffe für Zubereitungen für die orale Aufnahme.

13. Verwendung der Mischungen nach Anspruch 1 als Löslichkeitsverbesserer für andere Aromastoffe.

## Claims

1. Substance mixtures, comprising
(a) steviol glycosides,
(b) starch degradation products and
(c) one or more phenolic sweetness enhancing flavouring agents selected from the group consisting of Hesperetin, Phloretin, 1-(2,4-Dihydroxyphenyl)-3-(3-hydroxy-4-methoxy-phenyl)-propan-I-on, 7,3-Dihydroxy-4'-methoxy-flavane and 5-Hydroxy-4-(4-hydroxy-3-methoxy-phenyl)-7-methoxy-2-chromanone,
wherein the substance mixtures contain the components (a+b) and (c) in a weight ratio of 1:99 to 99:1.

2. Substance mixtures according to claim 1, **characterized in that** the steviol glycosides have 2 to 6 glucose units.

3. Substance mixtures according to claims 1 and/or 2, **characterized in that** the steviol glycosides contain
(a1) 30 to 50 wt.-% Rebaudioside A,
(a2) 5 to 10 wt.-% Rebaudioside B,
(a3) 5 to 10 wt.-% Rebaudioside C,
(a4) 20 to 30 wt.-% Rebaudioside D,
(a5) 15 to 25 wt.-% steviosides and
(a6) 1 to 5 wt.-% steviol biosides,
with the proviso that the amounts add to 100 wt.-%.

4. Substance mixtures according to at least one of claims 1 to 3, **characterized in that** the starch degradation products are dextrins.

5. Substance mixtures according to at least one of claims 1 to 4, **characterized in that** the substance mixtures contain the components (a) and (b) in a weight ratio of 60:40 to 90:10.

6. Compositions for oral intake, comprising the mixtures according to claim 1.

7. Compositions according to claim 6, **characterized in that** the compositions contain 0.00001 to 2 wt.-% of the mixture according to claim 1.

8. Compositions according to claims 6 and/or 7, **characterized in that** the compositions are selected from the group consisting of baked goods, confectionery, alcoholic or non-alcoholic drinks, fruit or vegetable juice compositions, instant drinks, meat products, eggs or egg products, grain products, dairy products, products made of soy protein or other soy bean fractions, products made of other vegetable protein sources, vegetable compositions, snacks, products on a fat and oil basis or emulsions thereof, other finished-products and soups, spices, seasoning mixes as well as particularly seasonings to be sprinkled.

9. Compositions according to claims 6 and/or 7, **characterized in that** the compositions are selected from the group consisting of oral care and dental care agents, mouthwashes and chewing gums.

10. Compositions according to at least one of the claims 6 to 9, **characterized in that** the compositions further comprise sweeteners, food acids, acidity regulators, thickeners and additional flavouring agents.

11. Method for optimizing the taste qualities of compositions for oral intake and for improving the solubility of flavouring agents in compositions for oral intake, **characterized in that** 0.0001 to 2 wt.-% of the mixtures according to claim 1 are added to the compositions.

12. Use of the mixtures according to claim 1 as flavouring agents for compositions for oral intake.

13. Use of the mixtures according to claim 1 as solubility improver for other flavouring agents.

## Revendications

1. Mélanges de substances contenant
(a) des glycosides de stéviol,
(b) des produits de dégradation de l'amidon et
(c) une ou plusieurs substances aromatisantes phénoliques intensifiant le goût sucré qui sont choisies dans le groupe constitué par l'hespérétine, la phlorétine, la 1-(2,4-dihydroxyphényl)-3-(3-hydroxy-4-méthoxy-phényl)propane-1-one, le 7,3-dihydroxy-4'-méthoxy-flavane et la 5-hydroxy-4-(4-hydroxy-3-méthoxy-phényl)-7-méthoxy-2-chromanone
les mélanges de substances contenant les composants (a+b) et (c) dans un rapport pondéral de 1 : 99 à 99 : 1.

2. Mélanges de substances selon la revendication 1, **caractérisés par le fait que** les glycosides de stéviol présentent entre 2 et 6 unités de glucose.

3. Mélanges de substances selon les revendications 1 et/ou 2, **caractérisés par le fait que** les glycosides de stéviol contiennent
(a1) entre 30 et 50 % en poids de rebaudioside A,
(a2) entre 5 et 10 % en poids de rebaudioside B,
(a3) entre 5 et 10 % en poids de rebaudioside C,
(a4) entre 20 et 30 % en poids de rebaudioside D,
(a5) entre 15 et 25 % en poids de stéviosides et
(a6) entre 1 et 5 % en poids de stéviolbiosides
à condition que les indications de quantité se complètent pour donner 100 % en poids.

4. Mélanges de substances selon l'une au moins des revendications 1 à 3, **caractérisés par le fait que** les produits de dégradation de l'amidon représentent des dextrines.

5. Mélanges de substances selon l'une au moins des revendications 1 à 4, **caractérisés par le fait qu'**ils contiennent les composants (a) et (b) dans un rapport pondéral de 60 : 40 à 90 : 10.

6. Préparations pour l'ingestion par voie orale, contenant les mélanges selon la revendication 1.

7. Préparations selon la revendication 6, **caractérisées par le fait qu'**elles contiennent entre 0,00001 et 2 % en poids du mélange selon la revendication 1.

8. Préparations selon les revendications 6 et/ou 7, **caractérisées par le fait qu'**elles sont choisies dans le groupe qui est constitué par les produits de boulangerie, les sucreries, les boissons alcoolisées ou non alcoolisées, les préparations à base de jus de fruits ou de légumes, les boissons instantanées, les produits à base de viande, les oeufs ou les produits à base d'oeufs, les produits à base de céréales, les produits laitiers, les produits faits de protéine de soja ou d'autres fractions de fève de soja, les produits d'autres sources de protéines végétales, les préparations à base de légumes, les biscuits apéritifs, les produits à base de graisse et d'huile ou les émulsions de ceux-ci, d'autres plats cuisinés et soupes, les épices, les mélanges d'épices ainsi que, en particulier, les assaisonnements.

9. Préparations selon les revendications 6 et/ou 7, **caractérisées par le fait qu'**elles sont choisies dans le groupe constitué par les produits de soins bucco-dentaires, les eaux dentifrices et les chewing-gums.

10. Préparations selon l'une au moins des revendications 6 à 9, **caractérisées par le fait qu'**elles contiennent en outre des édulcorants, des acides alimentaires, des régulateurs de l'acidité, des épaississants et des substances aromatisantes supplémentaires.

11. Procédé d'optimisation de la saveur de préparations pour l'ingestion par voie orale et pour améliorer la solubilité de substances aromatisantes dans des préparations pour l'ingestion par voie orale, **caractérisé par le fait que** l'on ajoute à celles-ci entre 0,0001 et 2 % en poids des mélanges selon la revendication 1.

12. Utilisation des mélanges selon la revendication 1 en tant que substances aromatisantes pour des préparations pour l'ingestion par voie orale.

13. Utilisation des mélanges selon la revendication 1 en tant qu'améliorants de solubilité pour d'autres substances aromatisantes.
